(19) Europäisches Patentamt
European Patent Office
Office européen des brevets

(11) Publication number: 0 123 741 A1

(12) **EUROPEAN PATENT APPLICATION**

(21) Application number: **83302366.6**

(22) Date of filing: **26.04.83**

(51) Int. Cl.³: **C 07 D 277/48**
// C07D277/56, C07C69/63, A61K31/425

(43) Date of publication of application: **07.11.84**
**Bulletin 84/45**

(84) Designated Contracting States: **CH DE FR IT LI NL**

(71) Applicant: **SHIONOGI & CO., LTD., 12, Dosho-machi 3-chome Higashi-ku, Osaka 541 (JP)**

(72) Inventor: **Hirai, Kentaro, 720, Uematsu-cho Teramachidori-Matsubara-sagaru, Shimogyo-ku Kyoto (JP)**

(74) Representative: **Bizley, Richard Edward et al, BOULT, WADE & TENNANT 27 Furnival Street, London EC4A 1PQ (GB)**

(54) **2-Guanidino-4-hydroxymethylthiazole and derivatives thereof.**

(57) Compounds of the formula:

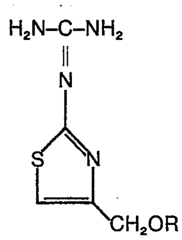

in which R is hydrogen, $C_1$–$C_5$ alkanoyl or substituted sulfonyl, which are useful as synthetic intermediates to 2-guanidino-4-[2-(formamido)ethylthiomethyl]thiazole, a histamine $H_2$ receptor antagonist. Methods of making the compounds are also provided.

EP 0 123 741 A1

## 2-GUANIDINO-4-HYDROXYMETHYLTHIAZOLE AND DERIVATIVES THEREOF

The present invention relates to 2-guanidino-4-hydroxymethylthiazole and derivatives thereof. More particularly, this invention relates to contents of the formula:

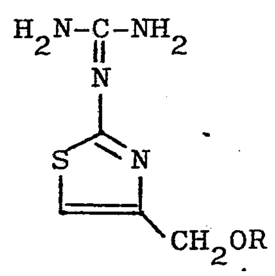

(I)

(in which R is hydrogen, $C_1$-$C_5$ alkanoyl or substituted sulfonyl)

which is useful as a synthetic intermediate to 2-guanidino-4-[2-(formamido)ethylthiomethyl]thiazole, histamine $H_2$ receptor antagonist. [U.S. pat. appln. No. 256,918].

In the formula (I), the $C_1$-$C_5$ alkanoyl includes illustratively formyl, acetyl, propionyl, butyryl and valeryl, and acetyl is preferred. The substituted sulfonyl includes illustratively $C_1$-$C_5$ alkanesulfonyl such as methanesulfonyl, ethanesulfonyl, propanesulfonyl,

butanesulfonyl and pentanesulfonyl, preferably methanesulfonyl (mesyl) and $C_6$-$C_8$ arenesulfonyl such as benzenesulfonyl, toluenesulfonyl, or xylenesulfonyl, preferably 4-toluenesulfonyl (tosyl).

The compounds of formula (I) can be prepared by the following scheme:

Route A

Route B

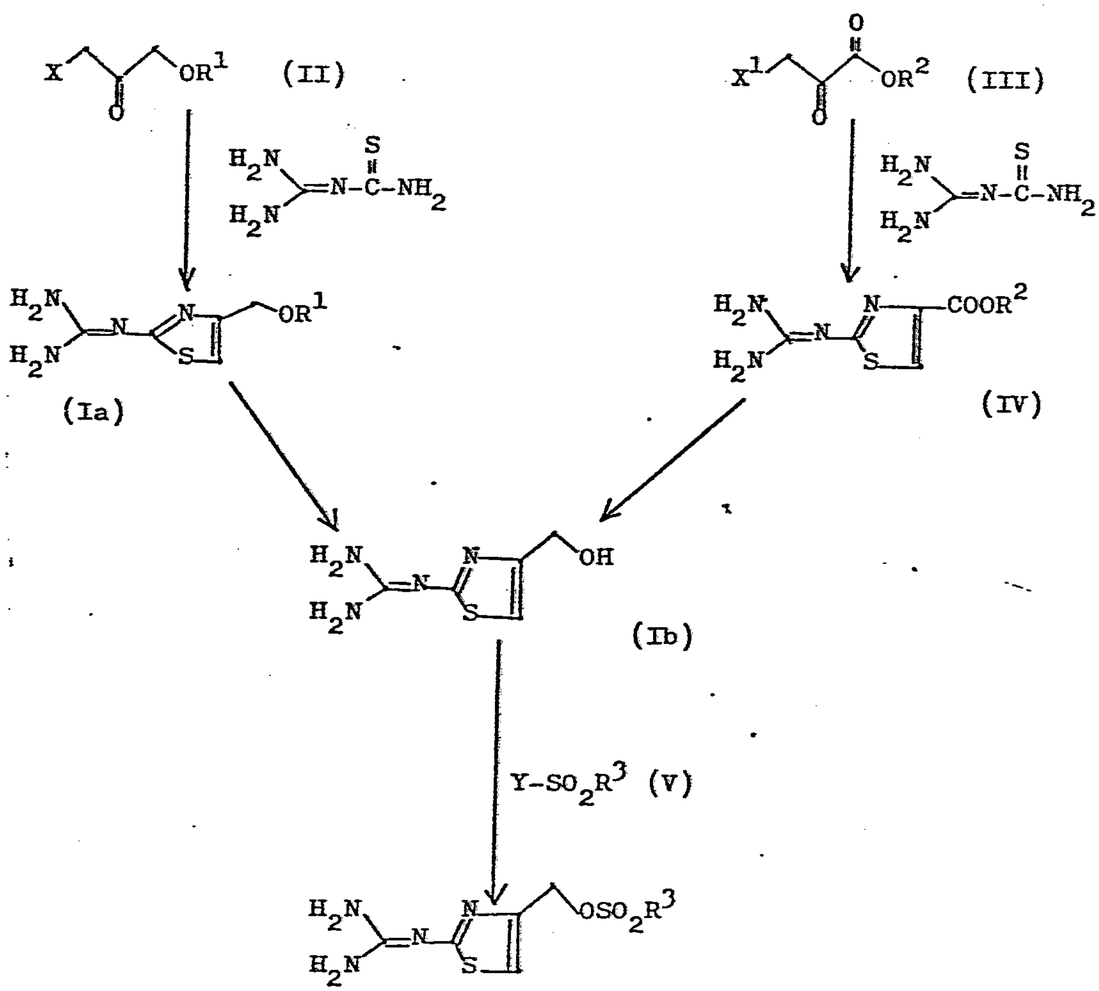


(II) (III) (Ia) (IV) (Ib) (Ic)

(in which $R^1$ is $C_1$-$C_5$ alkanoyl, $R^2$ is $C_1$-$C_5$ alkyl, $R^3$ is $C_1$-$C_5$ alkyl or $C_6$-$C_8$ aryl, and X, $X^1$ and Y are each independently halogen (e.g. chlorine, bromine, etc.)).

Route A

2-Guanidino-4-(alkanoyloxymethyl)thiazole (Ia) is prepared by reacting 1-alkanoyloxy-3-halo-2-propanone (II) with guanylthiourea in an inert solvent such as acetone,

dimethylformamide, dimethylsulfoxide, or pyridine. The reaction may be performed at room temperature or under heating at up to 100°C, preferably 20 to 70°C.

Then the above product (Ia) is subjected to hydrolysis with an inorganic base such as alkali metal hydroxide (e.g. sodium hydroxide, potassium hydroxide), alkali metal hydrogencarbonate (e.g. potassium hydrogencarbonate, sodium hydrogencarbonate) or alkali metal carbonate (e.g. potassium carbonate, sodium carbonate). The reaction may be performed at room temperature (about 15 to 20°C) or under heating at up to 100°C in a solvent such as methanol, ethanol, dioxane, acetone, dimethylsulfoxide or water or a mixed solvent thereof. Thus 2-guanidino-4-hydroxymethylthiazole (Ib) is prepared in a good yield.

The starting 1-alkanoyloxy-3-halo-2-propanone (II) is prepared by reacting epihalohydrin with an alkanoic acid to give 1-alkanoyloxy-3-halo-2-propanol and oxidizing the resulting propanol with a Jones reagent.

Route B

2-Guanidino-4-alkoxycarbonylthiazole (IV) is prepared by reacting alkyl 3-halopyruvate (III) with guanylthiourea in a solvent. The reaction is performed under heating at about 50 to 120°C, preferably about 65 to 95°C in a solvent such as methanol, ethanol, dimethylformamide, dimethylsulfoxide, pyridine or hexamethylphosphoric triamide.

Then the above product (IV) is reduced with an aluminum hydride compound such as lithium aluminum hydride,

sodium bis(methoxyethoxy)aluminum hydride or lithium bis(methoxyethoxy)aluminum hydride. The reaction is performed at room temperature to the boiling point of the solvent, in an appropriate solvent, for instance tetrahydrofuran, dioxane, diglyme or toluene.

Thus 2-guanidino-4-hydroxymethylthiazole (Ib) is prepared in a good yield.

The above product (Ib) is converted into 2-guanidino-4-(substituted sulfonyloxymethyl)thiazole (Ic) by reacting the compound (Ib) with an alkanesulfonyl or arenesulfonyl halide (V) in the presence of a base such as pyridine or triethylamine. The reaction may be performed in a solvent such as acetone, toluene, dimethylformamide, dimethylsulfoxide, or hexamethylphosphoric triamide at room temperature.

The product (I), namely 2-guanidino-4-hydroxymethylthiazole (Ib), and derivatives thereof (Ia) and (Ic) are useful as synthetic intermediates to 2-guanidino-4-[2-(formamido)ethylthiomethyl]thiazole, histamine $H_2$ receptor antagonist [U.S. pat. appln. No. 256,918]. For example, said $H_2$ antagonist is prepared by reacting 2-guanidino-4-hydroxymethylthiazole (Ib) with N,N'-diformylcystamine in the presence of a phosphorus compound such as tributylphosphine, triphenylphosphine or methylphenylaminotriphenylphosphonium iodide [J.Org.Chem.,<u>28</u>, 483 (1963)]. This reaction may be performed under cooling or heating (0° to 100°C), preferably at room temperature in a protic solvent such as pyridine, dimethylformamide or the like.

Presently preferred and practical embodiments of the present invention are illustratively shown in the following examples.

Example 1.

Preparation of 2-guanidino-4-acetoxymethylthiazole hydrochloride:

1) A suspension of epichlorhydrin (27.8 g) and sodium acetate (12.3 g) in acetic acid (36 g) is heated at 50-55°C for 8 hours. The reaction mixture is allowed to stand overnight and concentrated in vacuum. The residue is neutralized with aqueous sodium hydrogencarbonate and shaken with ether. The ethereal layer is dried over anhydrous sodium sulfate and concentrated in vacuum. The residue is distilled in vacuum to give 1-acetoxy-3-chloro-2-propanol (30 g) as an oil boiling at 115-116°C/13 mm Hg. The yield is 65.4 %.

2) To a solution of 1-acetoxy-3-chloro-2-propanol (15.3 g) in acetone (250 ml) is dropwise added Jones reagent (37.4 ml) containing chromic anhydride (10.39 g) and conc. sulfuric acid (10.18 ml) in 1 hour, and the resultant mixture is stirred at room temperature for 3 hours. The reaction mixture is mixed with isopropanol (10 ml) and the insoluble material is filtered off. The organic layer is concentrated in vacuum, and the residue is distilled to give 1-acetoxy-3-chloro-2-propanone (9 g) as an oil boiling at 102-104°C/15 mm Hg. The yield is 60.0 %.

NMR ($CDCl_3$), δ 2.17 (s, 3H),
4.13 (s, 2H),
4.87 (s, 2H).

[Hesse, et al., Ber. 48, 1986 (1915)].

3) A suspension of 1-acetoxy-3-chloro-2-propanone (4.52 g) and guanylthiourea (3.55 g) in acetone (100 ml) is stirred at room temperature for 64 hours. The precipitated crystals are filtered to give 2-guanidino-4-acetoxymethylthiazole hydrochloride hemihydrate (6.2 g) as crystals melting at 170-172°C.

Example 2.

Preparation of 2-guanidino-4-hydroxymethylthiazole:

1) A suspension of guanylthiourea (2.36 g) and ethyl 3-bromopyruvate (4.8 g) in dry ethanol (50 ml) is refluxed under stirring for 2.5 hours. The reaction mixture is concentrated in vacuum to dryness, and the residue is dissolved in water (about 100 ml). The aqueous solution is washed with ethyl acetate and made alkaline with 5 % sodium hydrogencarbonate solution. The precipitated crystals are filtered, washed with water and dried to give 2-guanidino-4-ethoxycarbonylthiazole (3.6 g) as crystals. The yield is 84 %.

NMR ($d_6$-DMSO), δ 1.28 (t, 3H, J=7 Hz),
4.23 (q, 2H, J=7 Hz),
6.93 (br. s, 4 H),
7.55 (s, 1 H).

2) To a suspension of lithium aluminum hydride (0.5 g) in dry tetrahydrofuran (260 ml) is portionwise added 2-guanidino-4-ethoxycarbonylthiazole (3.6 g) at 0°C with stirring in half an hour, and the resultant mixture is refluxed under stirring for 4 hours. Ethyl acetate and water are addded to the reaction mixture for decomposing an excess lithium aluminum hydride. The organic layer is separated and the residual layer is extracted with methanol. The methanolic extract is combined with the organic layer, dried over anhydrous sodium sulfate and concentrated in vacuum. The residue is chromatographed on a column of silica gel, which is eluted with ethyl acetate: methanol (4:1, v/v). The eluate is concentrated in vacuum to give 2-guanidino-4-hydroxymethylthiazole (1.8 g) as crystals melting at 163-165°C. The yield is 62.2 %.

NMR ($CD_3OD$), δ 4.48 (s, 2 H),
6.57 (s, 1 H).

<u>Example 3.</u>

Preparation of 4-guanidino-2-hydroxymethylthiazole:

To a solution of sodium hydroxide (2 g) in methanol (100 ml) is added 2-guanidino-4-acetoxymethylthiazole hydrochloride hemihydrate (6.15 g), and the resultant mixture is stirred at room temperature for half an hour. The insoluble material is filtered off, and the filtrate is concentrated in vacuum. The residue is chromatographed on a column of silica gel, which is eluted with methanol: ethyl acetate (1: 4, v/v). The eluate is concentrated

to give 2-guanidino-4-hydroxymethylthiazole (3.5 g) as crystals melting at 163 to 165°C. The yield is 82.9 %.

Anal. Calcd. for $C_5H_8N_4OS$: C, 34.87; H, 4.68; N, 32.54; S, 18.62 (%). Found: C, 34.88; H, 4.58; N, 32.13; S, 18.51 (%).

Example 4.

Preparation of 2-guanidino-4-hydroxymethylthiazole:

Using potassium carbonate (6.6 g) and methanol (90 ml), the reaction is performed as in Example 3, whereby 2-guanidino-4-hydroxymethylthiazole (7.2 g) is obtained from 2-guanidino-4-acetoxymethylthiazole hydrochloride hemihydrate (12.4 g). The yield is 87.2 %.

Example 5.

Preparation of 2-guanidino-4-(4-toluenesulfonyloxymethyl)thiazole:

To a solution of 2-guanidino-4-hydroxymethylthiazole (0.69 g) in pyridine (5 ml) is gradually added 4-toluenesulfonyl chloride (0.763 g), and the resultant mixture is stirred at room temperature for 4 hours. The resultant mixture is concentrated in vacuum, and the residue is chromatographed on a column of silica gel, which is eluted with methanol. The eluate is concentrated in vacuum to give 2-guanidino-4-(4-toluenesulfonyloxymethyl)-thiazole (0.6 g) as an oil. The yield is 45.8 %.

NMR ($CD_3OD$), δ 2.33 (s, 3 H),
5.87 (s, 2 H),
7.62 (s, 1 H),
7.17-7.66 (ABq, J=8 Hz, 4 H),

IR $\nu_{max}^{film}$ 1170, 1220 $cm^{-1}$

Example 6.

To a solution of 2-guanidino-4-hydroxymethylthiazole (0.86 g) in pyridine (6 ml) is gradually added methanesulfonyl chloride (0.6 g) under ice cooling, and the resultant mixture is stirred at room temperature for 2 hours. The reaction mixture is concentrated in vacuum, and the residual oil is chromatographed on a column of silica gel, which is eluted with methanol. The eluate is concentrated in vacuum to give 2-guanidino-4-(methanesulfonyloxymethyl)-thiazole (1.2 g) as an oil. The yield is 96 %.

NMR ($CD_3OD$), δ 2.70 (s, 3 H),
5.83 (s, 2 H),
7.57 (s, 1 H).

IR $\nu_{max}^{film}$ 1170, 1220 $cm^{-1}$

Reference Example

A mixture of 2-guanidino-4-hydroxymethylthiazole (0.517 g), N,N'-diformylcystamine (1.87 g), tributylphosphine (1.82 g) and pyridine (1.5 ml) is stirred at room temperature for 8 hours. The reaction mixture is allowed to stand overnight and concentrated in vacuum and treated with maleic acid. The residue is chromatographed on a column of silica gel, which is eluted with methanol:ethyl acetate (1:4, v/v). The eluate is concentrated in vacuum to give 2-guanidino-4-[2-(formamido)ethylthiomethyl]thiazole maleate (0.81 g) as crystals melting at 146-148°C. The yield is 71.7 %.

CLAIMS:

1. A compound of the formula:

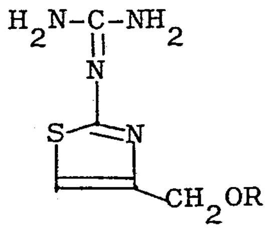

in which R is hydrogen, $C_1-C_5$ alkanoyl, or substituted sulfonyl.

2. A compound as claimed in claim 1, in which R is hydrogen.

3. A compound as claimed in claim 1, in which R is $C_1-C_5$ alkanoyl.

4. A compound as claimed in claim 3, in which R is acetyl.

5. A compound as claimed in claim 1, in which R is $C_1-C_5$ alkanesulfonyl.

6. A compound as claimed in claim 5, in which R is methanesulfonyl.

7. A compound as claimed in claim 1, in which R is $C_6-C_8$ arenesulfonyl.

8. A compound as claimed in claim 7, in which R is toluenesulfonyl.

9. A compound as claimed in claim 7, in which the toluenesulfonyl is 4-toluenesulfonyl.

10. A method of making a compound as claimed in

claim 1, and wherein R is $C_1$-$C_5$ alkanoyl, hydrogen or substituted sulfonyl, the method comprising (a) reacting a 1-alkanoyloxy-3-halo-2-propanone with guanylthiourea to form a 2-guanidino-4-($C_1$-$C_5$-alkanoyloxymethyl) thiazole, then if desired (b) hydrolyzing the 2-guanidino-4-($C_1$-$C_5$-alkanoyloxymethyl) thiazole with a base to form 2-guanidino-5-hydroxymethylthiazole, and then if desired (c) reacting the 2-guanidino-4-hydroxylmethylthiazole with a substituted sulfonyl halide in the presence of a base to form a 2-guanidino-4-(substituted sulfonyloxymethyl) thiazole.

11. A method as claimed in claim 10 wherein step (a) is carried out in an inert solvent.

12. A method as claimed in claim 10 or claim 11 wherein step (a) is carried out at a temperature of from room temperature to 100°C.

13. A method as claimed in any one of claims 10 to 12 wherein in step (b) the base is an inorganic base.

14. A method as claimed in claim 13 wherein step (b) is carried out at a temperature of from room temperature to 100°C.

15. A method of making a compound as claimed in claim 1 and wherein R is $C_1$-$C_5$ alkyl, hydrogen or substituted sulfonyl, the method comprising (a) reacting a $C_1$-$C_5$ alkyl 3-halopyruvate with guanylthiourea to form

a 2-guanidino-4-$C_1$-$C_5$ alkoxycarbonylthiazole, then if desired (b) reducing the 2-guanido-4-alkoxycarbonylthiazole with an aluminium hydride compound to form 2-guanidino-4-hydroxymethylthiazole, and then if desired (c) reacting the 2-guanidino-4-hydroxymethylthiazole with a substituted sulfonyl halide in the presence of a base to form a 2-guanidino-4-(substituted sulfonyloxymethyl) thiazole.

16. A method as claimed in claim 15 wherein step (a) is carried out at a temperature of from 50 to 120°C.

17. A method as claimed in claim 15 or claim 16 wherein step (a) is carried out in the presence of a solvent.

18. A method as claimed in any one of claims 15 to 17 wherein step (b) is carried out in an inert solvent.

19. A method as claimed in claim 18 wherein step (b) is carried out at a temperature of from room temperature to the boiling point of the solvent.

20. A method as claimed in any one of claims 10 to 19 wherein step (c) is carried out in a solvent.

21. A method as claimed in any one of claims 10 to 20 wherein step (c) is carried out at room temperature.

22. A method of making a compound as claimed in claim 1 wherein R is hydrogen or substituted sulfonyl, the method comprising step (b) and/or step (c) of any one

of claims 10, 13 to 15 or 18 to 21.

23. 2-guanidino-4-[2-(formamido)ethylthiomethyl] thiazole whenever made from a compound as claimed in any one of claims 1 to 9 or by a method comprising a method as claimed in any one of claims 10 to 22.

24. A compound as claimed in any one of claims 1 to 9 intended for the preparation of 2-guanidino-4-4[2-(formamido)ethylthiomethyl]thiazole.

European Patent Office

# EUROPEAN SEARCH REPORT

0123741

Application number

EP 83 30 2366

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,Y | GB-A-2 076 397 (SHIONOGI) * The whole patent application, particularly page 2 formula IV * | 1-24 | C 07 D 277/48 // C 07 D 277/56 C 07 C 69/63 A 61 K 31/425 |
| X,Y | US-A-4 165 378 (D.J. GILMAN) * The wole patent, particularly column 5, formula VIII * | 1-24 | |
| Y | DE-A-3 033 169 (BRISTOL-MEYERS) * Page 118,119, example 43B * | 1-24 | |
| | | | TECHNICAL FIELDS SEARCHED (Int. Cl. 3) |
| | | | C 07 D 277/00 |

The present search report has been drawn up for all claims

| Place of search | Date of completion of the search | Examiner |
|---|---|---|
| THE HAGUE | 15-12-1983 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82